# EUROPÄISCHE PATENTSCHRIFT

(11) **EP 2 066 382 B1**
(45) Veröffentlichungstag und Bekanntmachung des Hinweises auf die Patenterteilung: **01.01.2014**
(21) Anmeldenummer: 07801720.9
(22) Anmeldetag: 17.08.2007
(51) Int. Cl.: A61M 15/00, G01F 11/18

(54) **KOLBENDOSIERER**
PISTON DOSING PUMP
DOSEUR À PISTON

(30) Priorität: 20.09.2006 DE 102006044752
(43) Veröffentlichungstag der Anmeldung: 10.06.2009
(73) Patentinhaber: Boehringer Ingelheim International GmbH, 55216 Ingelheim (DE)
(72) Erfinder: KLADDERS, Heinrich, 45468 Muelheim-Ruhr (DE)
(74) Vertreter: Simon, Elke Anna Maria
(86) Internationale Anmeldenummer: PCT/EP2007/007270
(87) Internationale Veröffentlichungsnummer: WO 2008/034506

(56) Entgegenhaltungen:
- EP-A- 0 516 510
- EP-A- 0 546 996
- DE-A1- 4 027 391
- US-A- 3 244 328
- US-A- 5 634 900

## Beschreibung

Die vorliegende Erfindung betrifft eine Dosiereinrichtung, vorzugsweise für einen Inhalator, zur Dosierung einer Kleinmenge einer insbesondere pulver-förmigen Formulierung nach dem Oberbegriff des Anspruchs 1 (vgl US 5634900 und EP0516510).

Die vorliegende Erfindung betrifft insbesondere einen Inhalator zur Ausgabe bzw. Inhalation einer vorzugsweise pulverförmigen Formulierung, also einen Pulverinhatator. Jedoch kann die Formulierung grundsätzlich auch in flüssiger Phase, als Dispersion oder in sonstiger fluidisierbarer Form vorliegen.

Bei der Formulierung handelt es sich insbesondere um ein therapeutisches Mittel bzw. Arzneimittel. Insbesondere enthält die Formulierung dementspre chend mindestens einen Wirkstoff oder besteht daraus. Die Formulierung dient also insbesondere der medizinischen Behandlung oder sonstigen thetapeutischen Zwecken.

Unter dem Begriff "Kleinmenge" ist bei der vorliegenden Erfindung vorzugsweise ein Volumen von etwa 0,1 bis 25 µl, insbesondere bis zu 10 µl, und/oder ein Gewicht von 0,1 bis 25 mg, insbesondere bis zu 10 mg, zu verstehen.

Die US 4,350,094 offenbart eine Dosiereinrichtung zur Dosierung einer Kleinmenge einer Formulierung. Eine Dosierkammer ist durch Wände aus porösem Material begrenzt und am Ende eines rohrförmigen Trägers angeordnet. Durch Unterdruck ist die zu dosierende Formulierung in die Dosierkannner saugbar. Durch Überdruck bzw. Druckluft kann die Formulierung aus der Dosierkammer wieder ausgetragen werden.

Die DE 40 27 391 A1 offenbart ein treibgasfreies Inhalationsgerät mit einer Dosierenrichtung zur Dosierung und Ausgabe einer pulverförmigen Formulierung. Die Dosierung erfolgt mittels eines Bands, das die Formulierung aus einer Dosierkammer in einen Inhalationskanal zieht. Die Formulierung wird dann über einen Frerndluflstrom ausgetragen, der vom Inhalationsgerät erzeugt wird.

Der vorliegenden Erfindung liegt die Aufgabe zugrunde, eine Dosiereinrichtung, vorzugsweise für einen Inhalator, einen Inhalator mit einer derartigen Dosiereinrichtung und ein Verfahren zur Dosierung einer Kleinmenge der Formulierung anzugeben, wobei ein einfacher kompakter Aufbau, eine einfache Bedienung, eine hochgradig genaue Dosierung und/oder eine optimale Anpaßbarkeit bzw. universelle Einsetzearkeit ermöglicht wird bzw. werden.

Die obige Aufgabe wird durch eine Dosiereinrichtung gemäß Anspruch 1 gelöst. Vorteilhafte Weiterbildungen sind Gegenstand der Unteransprüche.

Ein erster Aspekt der vorliegenden Erfindung liegt darin, mittels einer Pumpeinrichtung abwechselnd einen Unterdruck bzw. Luftstrom zur Aufnahme bzw. zum Einsaugen der Formulierung in die Dosierkammer und einen Überdruck bzw. Luftstrom zur Abgabe der Formulierung aus der Dosierkammer zu erzeugen. Dies gestattet einen einfachen, kompakten und kostengünstigen Aufbau und eine einfache Bedienung bzw. Betätigung.

Ein zweiter Aspekt der vorliegenden Erfindung legt darin, einen Kolben vorzusehen, der die Dosierkammer trägt bzw. bildet und der zwischen einer Auf nahmeposition zur Aufnahme der Formulierung und einer Abgabeposition zur Abgabe der Formulierung bewegbar ist. Dies gestattet bei einem einfachen und kompakten Aufbau eine optimierte Dosierung.

Unter dem Begriff "Kolben" ist insbesondere ein längliches oder stabförmiges, vorzugsweise zylindrisches Element zu verstehen. Jedoch ist in einem weiteren Sinne auch jedes sonstige bewegbare Element zu verstehen. Insbesondere ist das Element als Ventilelement oder Steuerelement ausgebildet.

Besonders bevorzugt erfolgt durch das Bewegen der Dosierkammer zwischen der Aufnahmeposition und der Abgabeposition und/oder durch das umgekehrte Bewegen eine Steuerung des Unterdrucks und/oder Überdrucks bzw. der Luftströme zur Aufnahme der Formulierung in die Dosierkammer und/oder Abgabe der Formulierung aus der Dosierkammer. Dies gestattet einen besonders einfachen Ablauf und einfachen, kompakten und kostengünstigen Aufbau.

Ein dritter Aspekt der vorliegenden Erfindung liegt darin, daß die Größe der Dosierkammer einstellbar ist. Dies ist bei einem einfachen Aufbau zur Erreichung einer gewünschten Dosierung vorteilhaft.

Die vorschlagsgemäße Dosiereinrichtung kann grundsätzlich für verschiedene Zwecke eingesetzt werden. Besonders bevorzugt weist ein Inhalator eine vorschlagsgemäße Dosiereinrichtung auf. Entsprechend wird ein einfacher, kompakter und kostengünstiger Aufbau der Inhalators bei großer Dosiergenauigkeit ermöglicht.

Bei der vorliegenden Erfindung sind die Begriffe "Luft" und "Luftstrom" vorzugsweise in einem weiteren Sinne auch dahingehend zu verstehen, daß auch ein sonstiges Gas bzw. eine Strömung eines sonstigen Gases umfaßt ist. Nachfolgend wird jedoch immer der Begriff "Luft" verwendet, da üblicherweise Luft als Gas und Fördermedium zur Dosierung und ggf. auch Förderung der Formulierung eingesetzt wird.

Weitere Aspekte, Merkmale, Eigenschaften und Vorteile der vorliegenden Erfindung ergeben sich aus den Ansprüchen und der folgenden Beschreibung bevorzugter Ausführungsformen anhand der Zeichnung. Es zeigt:
- Fig. 1: einen schematischen Schnitt eines Inhalators gemäß einer ersten Ausführungsform bei der Dosierung;
- Fig. 2: eine ausschnittsweise Vergrößerung von Fig. 1;
- Fig. 3: einen schematischen Schnitt des Inhalators gemäß Fig. 1 bei der Ausgabe;
- Fig. 4: einen ausschnittsweisen, schematischen Schnitt eines Inhalators gemäß einer zweiten Ausführungsform; und
- Fig. 5: einen ausschnittsweisen, schematischen Schnitt eines Inhalators gemäß einer dritten Ausführungsform.

In den Figuren werden für gleiche oder ähnliche Teile die gleichen Bezugszeichen verwendet, auch wenn eine wiederholte Beschreibung weggelassen ist. Insbesondere ergeben sich dann auch die gleichen oder entsprechende Vorteile und Eigenschaften. Die einzelnen Figuren sind aus Darstellungs- oder Vereinfachungsgründen nicht maßstabsgerecht und auf wesentliche für die Erfindung relevante Komponenten reduziert.

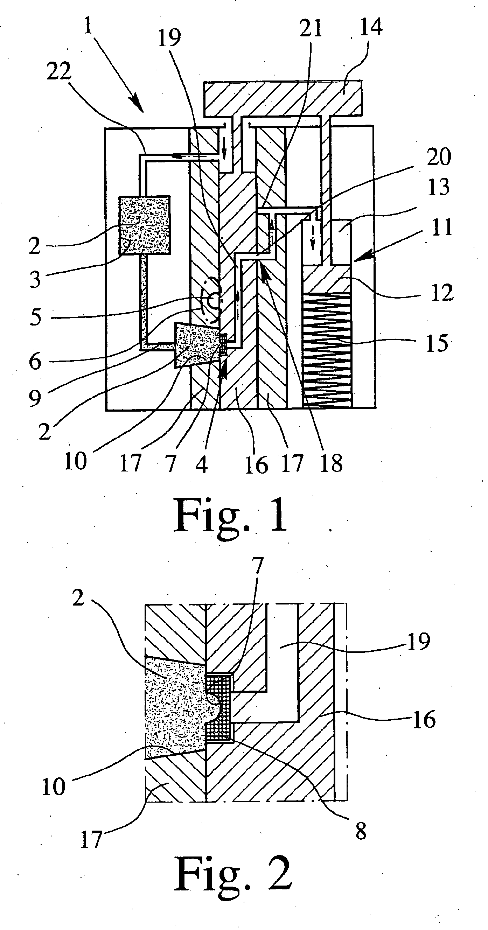

Fig. 1 zeigt schematisch den Aufbau eines vorschlagsgemäßen Inhalators 1 gemäß einer ersten Ausführungsform. Der Inhalator 1 ist vorzugsweise tragbar ausgebildet und/oder arbeitet insbesondere nur mechanisch.

Der Inhalator 1 dient der Ausgabe bzw. Inhalation einer vorzugsweise pulverförmigen Förmulierung 2 im eingangs genannten Sinne.

Beim Darstellungsbeispiel liegt die Formulierung 2 insbesondere als Schüttung bzw. lose vor. Insbesondere ist die Formulierung 2 in einem Reservoir 3 o. dgl. aufgenommen.

Der Inhalator 1 weist weiter eine vorschlagsgemäße Dosiereinrichtung 4 zur Dosierung der Formulierung 2 in Kleinmengen im oben genannten Sinne auf. Insbesondere erfolgt eine Dosierung nacheinander bzw. jeweils vor der nächsten Inhalation bzw. Ausgabe der Formulierung 2.

Die Dosiereinrichtung 4 bildet insbesondere einen Teil des Inhalators 1 oder ist in diesen integriert bzw. von diesem gebildet. Jedoch kann die Dosiereinrichtung 4 generell auch von dem Inhalator 1 trennbar sein. Des weiteren ist die vorschlagsgemäße Dosiereinrichtung 4 grundsätzlich auch für sonstige Zwecke, bei Meßvorrichtungen oder dergleichen einsetzbar.

Die zugemessene bzw. dosierte Formulierung 2 wird vorzugsweise über einen Ausgabekanal 5 und insbesondere einen sich daran anschließenden, nur strichpunktiert angedeuteten Auslaß 6, wie ein Mundstück oder dergleichen, ausgegeben bzw. ausgetragen.

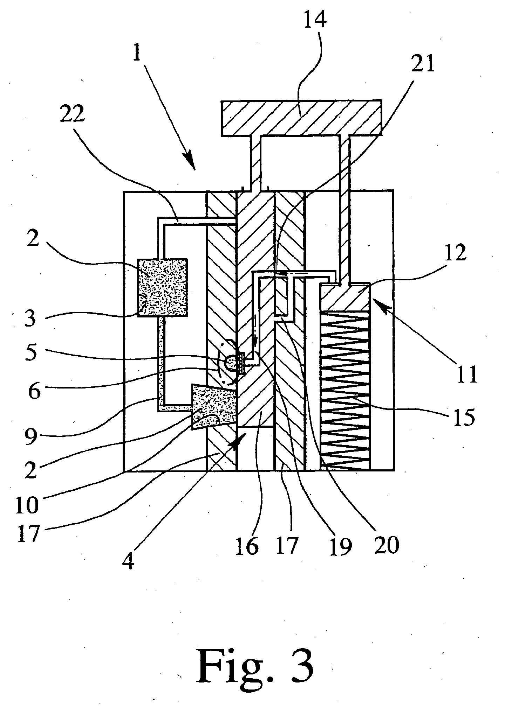

Fig. 1 zeigt den Inhalator 1 bzw. die Dosiereinrichtung 4 beim Zumessen bzw. Dosieren, insbesondere beim Füllen einer Dosierkammer 7 der Dosiereinrichtung 4. Fig. 2 zeigt in einer ausschnittsweisen Vergrößerung von Fig. 1 einen bevorzugten Aufbau der Dosierkammer 7 und veranschaulicht das Füllen der Dosierkammer 7. Fig. 3 zeigt in einem zu Fig. 1 korrespondierenden, schematischen Schnitt den Inhalator 1 bzw. die Dosiereinrichtung 4 bei der Ausgabe der Formulierung 2, insbesondere dem Leeren der Dosierkammer 7.

Die Dosierkammer 7 dient der Aufnahme bzw. dem Zumessen oder Dosieren der Formulierung 2. Die Dosierkammer 7 weist vorzugsweise eine zumindest bereichsweise gasdurchlässige Wandung 8 auf. Insbesondere besteht die Dosierkammer 7 bzw. Wandung 8 zumindest bereichsweise aus porösem Material, vorzugsweise Sintermaterial. Besonders bevorzugt ist die Dosierkammer 7 napf- bzw. topfförmig ausgebildet, wie in Fig. 2 angedeutet. Jedoch sind auch andere Konfigurationen möglich.

Beim Darstellungsbeispiel ist die Dosierkammer 7 bzw. Wandung 8 vorzugsweise durch einen Einsatz insbesondere aus porösem Material zumindest teilweise, vorzugsweise vollständig gebildet. Jedoch sind hier auch andere Anordnungen bzw. Aufbauten möglich. Beispielhaft wird hierzu auf die eingangs genannte US 4,350,049 verwiesen.

Die Formulierung 2 ist insbesondere durch Unterdruck aus dem Reservoir 3 in der Dosierkammer 7 bzw. einen Luftstrom in die Dosierkammer 7 aufnehmbar, insbesondere in diese einsaugbar. Beim Darstellungsbeispiel ist die Formulierung 2 vom Reservoir 3 über eine optionale Zuführleitung 9 und/oder einen Zwischenspeicher bzw. eine Zuführkammer 10 der Dosierkammer 7 zuführbar.

Das An- bzw. Einsaugen der Formulierung 2 durch Unterdruck und gegebenenfalls sogar Vakuum bzw. durch einen Luftstrom in die Dosierkammer 7 oder durch diese hindurch bewirkt ein besonders effektives und definiertes bzw. vollständiges Füllen der Dosierkammer 7 mit der Formulierung 2. Besonders bevorzugt wird der Unterdruck dadurch angelegt, daß durch die poröse Wandung 8 hindurch Luft aus der Dosierkammer 7 abgesaugt wird. Jedoch kann die Dosierkammer 7 zusätzlich oder alternativ auch durch Einwirkung der Gewichtskraft und/oder sonstiger Effekte mit der Formulierung 2 gefiillt werden.

Die Ausgabe der Formulierung 2 und/oder das Leeren der Dosierkammer 7 erfolgen vorzugsweise durch Überdruck bzw. einen Luftstrom aus der Dosierkammer 7. Dies ermöglicht ein sehr effektives, schnelles und definiertes bzw. vollständiges Leeren der Dosierkammer 7. Weiter kann hierdurch das Dispergieren der Formulierung 2 unterstützt werden. Insbesondere wird Druckluft bzw. der Luftstrom durch die poröse Wandung 8 in die Dosierkammer 7 geleitet. Zusätzlich oder alternativ kann das Leeren der Dosierkammer 7 auch durch die Gewichtskraft, eine Vibration, ein Schlagen oder sonstige Effekte unterstützt oder bewirkt werden.

Der Inhalator 1 bzw. die Dosiereinrichtung 4 weist eine Pumpeinrichtung 11 auf, die besonders bevorzugt abwechselnd sowohl den Unterdruck als auch den Überdruck bzw. die jeweiligen Luftströme erzeugt. Jedoch kann der Unterdruck bzw. Luftstrom zum Füllen der Dosierkammer 7 und/oder der Überdruck bzw. Luftstrom zum Leeren der Dosierkammer 7 auch von separaten Einrichtungen und/oder einer getrennten Einrichtung oder dergleichen bereitgestellt bzw. zugeführt werden.

Beim Darstellungsbeispiel weist die Pumpeinrichtung 11 einen Pumpenkolben 12 auf, der in einem Pumpenraum 13 hin- und herbewegbar ist, insbesondere aufgrund einer zugeordneten Betätigungseinrichtung 14 und/oder Feder 15. Insbesondere ist die Pumpeneinrichtung 11 bzw. Betätigungseinrichtung 14 manuell betätigbar. Die optionale Feder 15 dient beim Darstellungsbeispiel einer Rückstellung des Pumpenkolbens 12.

Beim Darstellungsbeispiel ist der Pumpenkolben 12 mittels der Betätigungseinrichtung 14 gegen die Kraft der Feder 15 in die in Fig. 1 gezeigte Stellung bewegbar, so daß im Pumpenraum 13 ein Unterdruck erzeugbar bzw. Luft in den Pumpenraum 13 einsaugbar ist. Bei der entgegengesetzten Bewegung des Pumpenkolbens 12 - insbesondere aufgrund der Rückstellkraft der Feder 15 nach Loslassen der Betätigungseinrichtung 14 bzw. einer davon gebildeten Handhabe oder Freigabe einer Sperre oder dergleichen - in die in Fig. 3 gezeigte Lage wird Luft aus dem Pumpenraum 13 verdrängt und dementsprechend ein Überdruck bzw. Luftstrom zum Leeren der Dosierkammer 7 erzeugt bzw. bereitgestellt. Jedoch sind hier auch andere technische Realisierungen möglich.

Beim Darstellungsbeispiel ist die Dosierkammer 7 zwischen einer Aufnahmeposition zur Aufnahme der Formulierung 2 (Fig. 1) und einer Abgabeposition zur Abgabe der Formulierung 2 (Fig. 3) und umgekehrt bewegbar. Diese Bewegung wird insbesondere zum Steuern des in der Dosierkammer 7 wirkenden Unterdrucks und/oder Überdrucks bzw. der entsprechenden Luftströme eingesetzt.

Vorzugsweise ist die Dosierkammer 7 an einem Kolben 16 im eingangs genannten Sinne angeordnet bzw. von diesem gebildet oder getragen. Der Kolben 16 ist zwischen der Aufnahmeposition und Abgabeposition hin- und herbewegbar. Besonders bevorzugt ist die Dosierkammer 7 bzw. der die Dosierkammer 7 bildende Einsatz oder dergleichen seitlich am Kolben 16 angeordnet und öffnet sich insbesondere zu einer den Kolben 16 zumindest partiell umgebenden Kolbenführung 17 oder dergleichen.

Der Kolben 16 ist vorzugsweise in der Kolbenführung 17 längs verschiebbar. Die Kolbenführung 17 ist mit der Zuführung für die Formulierung 2 - beim Darstellungsbeispiel mit der Zufiihrkammer 10 - versehen, so daß in der Aufnahmeposition (Fig. 1 und 2) des Kolbens 16 der Dosierkammer 7 die Formulierung 2 zuführbar ist, insbesondere die seitlich offene Dosierkammer 7 unmittelbar mit der dann benachbarten Zuführkammer 10 bzw. dem Reservoir 3 oder dergleichen in Verbindung steht.

In der Abgabeposition ist der Kolben 16 derart verschoben, daß die Dosierkammer 7 mit dem Ausgabekanal 5 in Verbindung steht (Fig. 3).

Beim Darstellungsbeispiel ist die Bewegung der Dosierkammer 7 von der Aufnahmeposition in die Abgabeposition bzw. umgekehrt insbesondere mit der Betätigung der Pumpeneinrichtung 11 gekoppelt, besonders bevorzugt dadurch, daß der Kolben 16 auch über die Betätigungseinrichtung 14 betätigbar bzw. bewegbar ist. Insbesondere ist der Kolben 16 hierzu fest mit der Betätigungseinrichtung 14 bzw. der davon gebildeten Handhabe verbunden, beim Darstellungsbeispiel ebenso wie der Pumpenkolben 12. Jedoch sind hier auch andere technische Lösungen möglich.

Die vorschlagsgemäße Dosiereinrichtung 4 bzw. Pumpeinrichtung 11 weist vorzugsweise eine Ventileinrichtung 18 auf zur Steuerung, wann die Dosierkammer 7 unter Unterdruck und/oder unter Überdruck gesetzt wird bzw. wann die Luft in die oder aus der Dosierkammer 7 gesaugt wird, um die Formulierung 2 in die Dosierkammer 7 zu fördern bzw. um die Formulierung 2 aus der Dosierkammer 7 auszutragen bzw. die Dosierkammer 7 zu leeren.

Die Ventileinrichtung 18 ist beim Darstellungsbeispiel vorzugsweise durch den Kolben 16 bzw. einen Verbindungskanal 19 im Kolben 16 und durch einen ersten Anschlußkanal 20 und einen zweiten Anschlußkanal 21 in der Kolbenführung 17 oder dergleichen gebildet. Die beiden Anschlußkanäle 20, 21 sind an den Pumpenraum 13 angeschlossen und längs der Kolbenführung 17 derart korrespondierend zu der Aufnahmeposition und Abgabeposition des Kolbens 16 bzw. der Dosierkammer 7 angeordnet oder versetzt, daß vorzugsweise nur in der Aufnahmeposition und der Abgabeposition jeweils eine pneumatische Verbindung zwischen dem jeweiligen Anschlußkanal 20 oder 21 einerseits und dem Verbindungskanal 19 andererseits hergestellt wird.

Mit dem vorgenannten Aufbau oder durch einen sonstigen Aufbau wird besonders bevorzugt der folgende Verfahrensablauf ermöglicht oder erreicht. Wenn die entleerte Dosierkammer 7 von der Abgabeposition in die Aufnahmeposition bewegt wird, wird im Pumpenraum 13 ein Unterdruck erzeugt. Wenn die Dosierkammer 7 dann die Aufnahmeposition erreicht, wird eine pneumatische Verbindung zwischen dem Verbindungskanal 19 und dem ersten Anschlußkanal 20 hergestellt, so daß - insbesondere schlagartig oder sehr schnell - der im Pumpenraum 13 und an den Anschlußkanälen 20, 21 herrschende Unterdruck über den Verbindungskanal 19 Luft aus der Dosierkammer 7 durch die poröse Wandung 8 hindurch einsaugt und dadurch - zumindest temporär - einen Unterdruck in der Dosierkammer 7 und/oder einen Luftstrom vom Reservoir 3 bzw. der Zuführkammer 10 in die Dosierkammer 7 erzeugt (dies ist in Fig. 1 durch Pfeile angedeutet), wodurch die Formulierung 2 in die Dosierkammer 7 gefördert bzw. in diese gesaugt wird. So wird die Dosierkammer 7 sehr schnell und definiert und vollständig mit der Formulierung 2 gefüllt oder dies zumindest unterstützt.

Beim Darstellungsbeispiel steht das Reservoir 3 bzw. die Zuführkammer 10 vorzugsweise über einen Zuluftkanal 22 mit der Umgebung in Verbindung, so daß über diesen Luft nachströmen kann und damit im Gleichgewicht wieder der Umgebungsdruck in der dann gefüllten Dosierkammer 7 hergestellt wird.

Grundsätzlich ist es jedoch auch möglich, daß die Pumpeinrichtung 11 das Reservoir 3 bzw. die Zuführkammer 10 mit der Formulierung 2 zum Füllen der Dosierkammer 7 mit der Formulierung 2 unter Überdruck setzt bzw. durch das Reservoir 3 und/oder die Zuführkammer 10 einen Luftstrom in die Dosierkammer 7 erzeugt, so daß die Formulierung 2 in der gewünschten Weise in die Dosierkammer 7 gefördert wird, wobei die zugeführte Luft über die poröse Wandung 8 der Dosierkammer 7 oder auf sonstige geeignete Weise entweichen kann. Auch hier wird also ein Luftstrom zum Füllen der Dosierkammer 7 mit der Formulierung 2 erzeugt.

Die gefüllte Dosierkammer 7 wird dann aus der Aufnahmeposition in die Abgabeposition bewegt. Im Verlauf dieser Bewegung wird die im Pumpenraum 13 befindliche Luft komprimiert, beim Darstellungsbeispiel der Pumpenkolben 12 nach oben in die in Fig. 3 gezeigte Position bewegt. Hierdurch wird ein Überdruck im Pumpenraum 13 bzw. den daran angeschlossenen Anschlußkanälen 20, 21 erzeugt. Erst bei Erreichen der Ausgabeposition öffnet die Ventileinrichtung 18, indem eine pneumatische Verbindung zwischen dem zweiten Anschlußkanal 21 und dem Verbindungskanal 19 hergestellt wird. Der Überdruck kann über die poröse Wandung 8 in die Dosierkammer 7 entweichen und dort einen Überdruck bzw. Luftstrom erzeugen, der die Formulierung 2 aus der Dosierkammer 7 austrägt, insbesondere in den sich anschließenden Ausgabekanal 5 des Inhalators 1. Dies ist in Fig. 3 insbesondere durch die Pfeile angedeutet.

Der das Leeren der Dosierkammer 7 bewirkende Luftstrom unterstützt insbesondere auch das Dispergieren der Formulierung 2 in sehr feine Partikel in den Ausgabekanal 5. Insbesondere erfolgt gleichzeitig mit oder unmittelbar nach dem Leeren der Dosierkammer 7 die Ausgabe der Formulierung 2 durch den Ausgabekanal 5 hindurch über den Auslaß 6. Insbesondere wird hierbei ein Luftstrom durch das Einatmen bzw. Inhalieren eines nicht dargestellten Benutzers erzeugt. Zusätzlich oder alternativ kann hierzu auch ein Luftstrom durch eine Pumpe oder sogar durch die Pumpeinrichtung 11 erzeugt werden. Alternativ oder zusätzlich kann das Inhalieren bzw. Einatmen eines Benutzers auch nur eine Auslösung der Ausgabe der Formulierung 2 bewirken, also eine sogenannte Atemzugtriggerung bilden.

Die vorschlagsgemäße Ausbildung gestattet einen sehr einfachen, kompakten und preisgünstigen Aufbau sowie eine sehr einfache Bedienung, insbesondere ist nur eine Betätigung der vorzugsweise gemeinsamen Betätigungseinrichtung 14 erforderlich.

Aufgrund des Füllens und Leerens der Dosierkammer 7 durch Unterdruck und Überdruck bzw. entsprechende Luftströme arbeitet die Dosiereinrichtung 4 bzw. der Inhalator 1 vorzugsweise zumindest im wesentlichen unabhängig von der Gewichtskraft bzw. jeweiligen räumlichen Orientierung, so daß in jeder Lage eine sehr genaue Dosierung ermöglicht wird.

Beim Darstellungsbeispiel bildet das Reservoir 3 vorzugsweise einen Teil der Dosiereinrichtung 4. Jedoch kann das Reservoir 3 auch getrennt davon ausgebildet und/oder auswechselbar sein.

Beim Darstellungsbeispiel ist die Pumpeinrichtung 11 vorzugsweise manuell betätigbar. Jedoch kann die Pumpeinrichtung 11 grundsätzlich auch auf sonstige Weise, beispielsweise elektrisch, betreibbar sein. Des weiteren ist der Begriff "Pumpeinrichtung" vorzugsweise in einem sehr weiten Sinne auch dahingehend zu verstehen, daß er auch jede sonstige Druckerzeugung und generell die Bereitstellung von unter Druck stehendem Gas oder dergleichen, beispielsweise durch einen Druckbehälter, eine Patrone oder dergleichen, umfaßt.

Die vorschlagsgemäße Dosiereinrichtung 4 kann insbesondere auch zum Füllen von Kapseln oder sonstigen Behältnissen bei einer insbesondere werkseitigen Dosierung der Formulierung 2 eingesetzt werden. Insbesondere werden dann die vordosierten Kapseln oder Behältnisse in einem üblichen Inhalator eingesetzt.

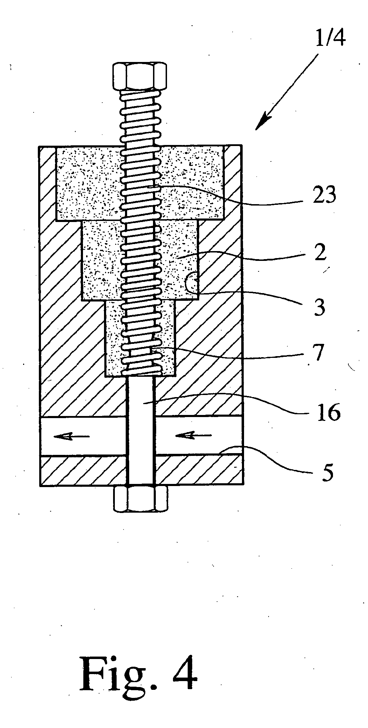

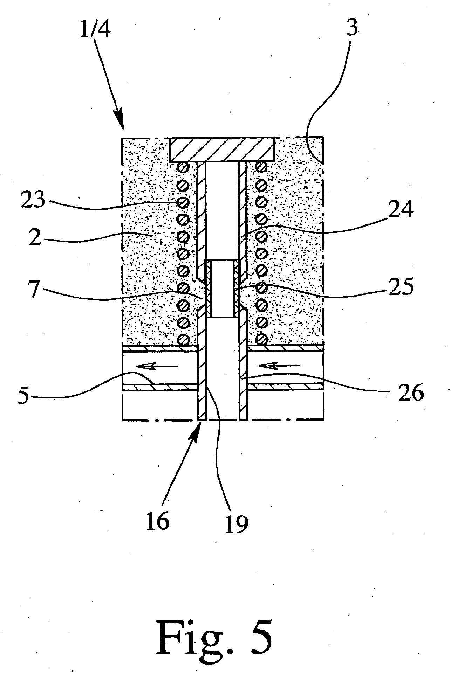

Nachfolgend werden weitere Ausführungsformen des vorschlagsgemäßen Inhalators 1 bzw. der vorschlagsgemäßen Dosiereinrichtung 4 anhand der nur ausschnittsweisen, sehr schematischen Darstellungen gemäß Fig. 4 und 5 erläutert. Die bisherigen Ausführungen und Erläuterungen gelten insbesondere entsprechend bzw. ergänzend. Jedoch können die nachfolgend beschriebenen Ausführungsformen auch unabhängig davon bei sonstigen Inhalatoren bzw. Dosiereinrichtungen oder dergleichen eingesetzt werden.

Fig. 4 zeigt eine zweite Ausführungsform in einem schematischen, ausschnittsweisen Schnitt. Der Kolben 16 mit der Dosierkammer 7 erstreckt sich hier vorzugsweise in das Reservoir 3 und insbesondere durch dieses hindurch.

Die Dosierkammer 7 ist hier insbesondere als umlaufende oder ringförmige Ausnehmung, Vertiefung oder Nut ausgebildet. Das Füllen der Dosierkammer 7 mit der Formulierung 2 erfolgt im Reservoir 3 vorzugsweise durch Gewichtskraft und/oder das Nachrutschen der vorzugsweise als lose Schüttung vorliegenden Formulierung 2.

Dem Kolben 16 ist vorzugsweise ein Mittel zur Lockerung der Formulierung 2 im Reservoir 3 zugeordnet. Das Mittel zur Lockerung verhindert insbesondere bei Bewegung des Kolbens 16, daß sich unerwünschte Brücken oder sonstige Verfestigungen der Formulierung 2 innerhalb des Reservoirs 3 bilden. Insbesondere führt das Mittel zur Lockerung bzw. zu einer gewissen Fluidisierung der Formulierung 2.

Beim Darstellungsbeispiel ist das Mittel zur Lockerung vorzugsweise durch eine Feder 23 gebildet. Besonders bevorzugt handelt es sich hierbei um eine Rückstellfeder 23 zur Rückstellung des Kolbens 16 in die Aufnahme- oder Ausgabeposition. Beim Darstellungsbeispiel spannt die Rückstellfeder 23 den Kolben 16 in die in Fig. 4 gezeigte Aufnahmeposition vor.

Der Kolben 16 ist aus der in Fig. 4 gezeigten Aufnahmeposition verschiebbar, so daß die Dosierkammer 7 aus dem Reservoir 3 in den zugeordneten Ausgabekanal 5 verlagert werden kann, wo die von der Dosierkammer 7 aufgenommene Formulierung 2 mittels eines durch Pfeile angedeuteten Luftstroms im eingangs genannten Sinne oder durch ein sonstiges geeignetes Fördermedium austragbar ist. Die Rückstellfeder 23 bewirkt dann vorzugsweise wieder eine Rückstellung in die Aufnahmeposition. Die hierbei erfolgende Bewegung (Kompression und Entspannung) der Rückstellfeder 23 bzw. eines sonstigen Elements in der Formulierung 2 und/oder relativ zum Kolben 16 bewirkt die gewünschte Lockerung bzw. Fluidisierung der Formulierung 2 im Reservoir 3.

Fig. 5 zeigt in einer ebenfalls schematischen, ausschnittsweisen Darstellung eine dritte Ausführungsform des vorschlagsgemäßen Inhalators 1 bzw. der vorschlagsgemäßen Dosiereinrichtung 4. Die dritte Ausführungsform entspricht in funktioneller Hinsicht im wesentlichen der zweiten Ausfiihrungsform, so daß auf die diesbezüglichen Ausführungen und Erläuterungen verwiesen wird. Nachfolgend werden lediglich wesentliche Unterschiede erläutert.

Bei der dritten Ausführungsform ist die Größe der Dosierkammer 7 einstellbar. Beim Darstellungsbeispiel wird dies dadurch ermöglicht, daß die Dosiereinrichtung 4 mindestens zwei gegeneinander - insbesondere durch Verschrauben oder Verdrehen - verstellbare Kolben- bzw. Dosierkammerteile 24 und 25 aufweist. Insbesondere sind die Teile 24 und 25 axial ineinander schraubbar. Beim Darstellungsbeispiel ist das Teil 25 insbesondere porös und/oder hülsenartig ausgebildet und bildet die gasdurchlässige Wandung 8 der Dosierkammer 7. Das Teil 25 ist von dem anderen Teil 24 bereichsweise überdeckt, wobei durch das Maß der Überdeckung die Größe - hier axiale Länge - der Dosierkammer 7 einstellbar ist.

Beim Darstellungsbeispiel ist das vorzugsweise gasdurchlässige Teil 25 am anderen Ende mit einem weiteren Kolben- bzw. Dosierkammerteil 26 verbunden oder daran angeformt. Insbesondere bilden die Teile 24 bis 26 den Kolben 16 und/oder begrenzen bzw. bilden die Dosierkammer 7.

Zur Einstellung der Größe der Dosierkammer 7 bzw. Verstellung sind insbesondere die Teile 24 und 25 bzw. die Teile 24 und 26 gegeneinander verdrehbar. Dies ermöglicht auf sehr einfache Weise eine Anpassung der Dosiereinrichtung 4 an die jeweils gewünschte Dosiermenge.

Bei der dritten Ausführungsform ist die Dosierkammer 7 bzw. der Kolben 16 vorzugsweise - zumindest bereichsweise - hohl ausgebildet, um das Füllen und Leeren der Dosierkammer 7 durch Unterdruck bzw. Überdruck und/oder entsprechende Luftströme unterstützen oder bewirken zu können. In diesem Fall ist insbesondere das hülsenartige bzw. innere Teil 25 porös ausgebildet, beispielsweise aus Sintermaterial hergestellt, um die gewünschte Gasdurchlässigkeit zu erreichen.

Beim Darstellungsbeispiel arbeitet der Inhalator besonders bevorzugt nur mechanisch. Grundsätzlich kann der Inhalator 1 jedoch auch elektrisch bzw. elektronisch arbeiten und/oder derartige Komponenten bzw. Bauteile enthalten. Dies gilt insbesondere für eine Triggereinrichtung zum Auslösen eines Austrags der Formulierung 2 oder der Inhalationsdauer, einen Antrieb, eine Pumpe, ein Zähleinrichtung, eine Sperre, eine Steuereinrichtung oder dergleichen.

Einzelne Merkmale und Aspekte der verschiedenen Ausführungsformen können auch beliebig miteinander kombiniert oder bei sonstigen Konstruktionen von Inhalatoren oder Dosiereinrichtungen eingesetzt werden.

Die vorliegende Erfindung ist nicht auf Inhalatoren beschränkt, sondern kann entsprechend auch bei sonstigen Zerstäubern eingesetzt werden. Dementsprechend ist der Begriff "Inhalator" vorzugsweise in einem weiteren Sinne auch dahingehend zu verstehen, daß er auch sonstige Spender oder Zerstäuber, insbesondere für medizinische oder sonstige therapeutische Zwecke, umfaßt.

Nachfolgend werden bevorzugte Bestandteile und/oder Zusammensetzungen der vorzugsweise medizinischen Formulierung 2 aufgeführt. Wie bereits erwähnt, handelt es sich insbesondere um Pulver, oder um Flüssigkeiten im weitesten Sinne. Besonders bevorzugt sind in der Formulierung 2 enthalten:

Die unten genannten Verbindungen können allein oder in Kombination zur Anwendung in der erfindungsgemäßen Vorrichtung gelangen. In den unten genannten Verbindungen ist **W** einen pharmakologisch, aktiver Wirkstoff und (beispielsweise) ausgewählt aus der Gruppe bestehend aus Betamimetika, Anticholinergika, Corticosteroiden, PDE4-Inhibitoren, LTD4-Antagonisten, EGFR-Hemmern; Dopamin-Agonisten, H1-Antihistaminika, PAF-Antagonisten und PI3-Kinase Inhibitoren. Weiterhin können zwei- oder dreifach Kombinationen von **W** kombiniert werden und zur Anwendung in der erfindungsgemäßen Vorrichtung gelangen. Beispielhaft genannte Kombinationen von **W** wären:
- **W** stellt ein Betamimetika dar, kombiniert mit einem Anticholinergika, Corticosteroide, PDE4-Inhibitore, EGFR-Hemmern oder LTD4-Antagonisten,
- **W** stellt ein Anticholinergika dar, kombiniert mit einem Betamimetika, Corticosteroiden, PDE4-Inhibitoren, EGFR-Hemmern oder LTD4-Antagonisten,
- **W** stellt ein Corticosteroiden dar, kombiniert mit einem PDE4-Inhibitoren, EGFR-Hemmem oder LTD4-Antagonisten
- **W** stellt ein PDE4-Inhibitoren dar, kombiniert mit einem EGFR-Hemmern oder LTD4-Antagonisten
- **W** stellt ein EGFR-Hemmern dar, kombiniert mit einem LTD4-Antagonisten.

Als Betamimetika gelangen hierbei vorzugsweise Verbindungen zur Anwendung, die ausgewählt sind aus der Gruppe bestehend aus Albuterol, Arformoterol, Bambuterol, Bitolterol, Broxaterol, Carbuterol, Clenbuterol, Fenoterol, Formoterol, Hexoprenaline, Ibuterol, Isoetharine, Isoprenaline, Levosalbutamol, Mabuterol, Meluadrine, Metaproterenol, Orciprenaline, Pirbuterol, Procaterol, Reproterol, Rimiterol, Ritodrine, Salmefamol, Salmeterol, Soterenol, Sulphonterol, Terbutaline, Tiaramide, Tolubuterol, Zinterol, CHF-1035, HO-KU-81, KUL-1248 und
- 3-(4-{6-[2-Hydroxy-2-(4-hydroxy-3-hydroxymethyl-phenyl)-ethylamino]-hexyloxy}-butyl)-benzyl-sulfonamid
- 5-[2-(5,6-Diethyl-indan-2-ylamino)-1-hydroxy-ethyl]-8-hydroxy-1H-quinolin-2-on
- 4-Hydroxy-7-[2-{[2-{[3-(2-phenylethoxy)propyl]sulphonyl}ethyl]-amino}ethyl]-2(3H)-benzothiazolon
- 1-(2-Fluor-4-hydroxyphenyl)-2-[4-(1-behzimidazolyl)-2-methyl-2-butylamino]ethanol
- 1-[3-(4-Methoxybenzyl-amino)-4-hydroxyphenyl]-2-[4-(1-benzimidazolyl)-2-methyl-2-butylamino]ethanol
- 1-[2H-5-hydroxy-3-oxo-4H-1,4-benzoxazin-8-yl]-2-[3-(4-N,N-dimethylaminophenyl)-2-methyl-2-propylamino]ethanol
- 1-[2H-5-hydroxy-3-oxo-4H-1,4-benzoxazin-8-yl]-2-[3-(4-methoxyphenyl)-2-methyl-2-propylamino]ethanol
- 1-[2H-5-hydroxy-3-oxo-4H-1,4-benzoxazin-8-yl]-2-[3-(4-n-butyloxyphenyl)-2-methyl-2-propylamino]ethanol
- 1-[2H-5-hydroxy-3-oxo-4H-1,4-benzoxazin-8-yl]-2-{4-[3-(4-methoxyphenyl)-1,2,4-triazol-3-yl]-2-methyl-2-butylamino}ethanol
- 5-Hydroxy-8-(1-hydroxy-2-isopropylaminobutyl)-2H-1,4-benzoxazin-3-(4H)-on
- 1-(4-Amino-3-chlor-5-trifluormethylphenyl)-2-tert.-butylamino)ethanol
- 6-Hydroxy-8-{1-hydroxy-2-[2-(4-methoxy-phenyl)-1,1-dimethyl-ethylamino]-ethyl}-4H-benzo[1,4]oxazin-3-on
- 6-Hydroxy-8-{1-hydroxy-2-[2-(4-phenoxy-essigsäureethylester)-1,1-dimethyl-ethylamino]-ethyl}-4H-benzo[1,4]oxazin 3-on
- 6-Hydroxy-8-{ 1-hydroxy-2-[2-(4-phenoxy-essigsäure)-1,1-dimethyl-ethylamino]-ethyl}-4H-benzo[1,4]oxazin-3-on
- 8-{2-[1,1-Dimethyl-2-(2,4,6-trimethylphenyl)-ethylamino]-1-hydroxy-ethyl}-6-hydroxy-4H-benzo[1,4]oxazin-3-on
- 6-Hydroxy-8-{1-hydroxy-2-[2-(4-hydroxy-phenyl)-1,1-dimethyl-ethylamino]-ethyl}-4H-benzo[1,4]oxazin-3-on
- 6-Hydroxy-8-{1-hydroxy-2-[2-(4-isopropyl-phenyl)-1,1 dimethyl-ethylamino]-ethyl}-4H-benzo[1,4]oxazin-3-on
- 8-{2-[2-(4-Ethyl-phenyl)-1,1-dirnethyl-ethylamino]-1-hydroxy-ethyl}-6-hydroxy-4H-benzo[1,4]oxazin-3-on
- 8-{2-[2-(4-Ethoxy-phenyl)-1,1-dimethyl-ethylamino]-1-hydroxy-ethyl}-6-hydroxy-4H-benzo[1,4]oxazin-3-on
- 4-(4-{2-[2-Hydroxy-2-(6-hydroxy-3-oxo-3,4-dihydro-2H-benzo[1,4]oxazin-8-yl)-ethylamino]-2-methyl-propyl}-phenoxy)-buttersäure
- 8-{2-[2-(3,4-Difluor-phenyl)-1,1-dimethyl-ethylamino]-1-hydroxy-ethyl}-6-hydroxy-4H-benzo[1,4]oxazin-3-on
- 1-(4-Ethoxy-carbonylamino-3-cyano-5-fluorophenyl)-2-(tert.-butylamino)ethanol
- 2-Hydroxy-5-(1-hydroxy-2-{2-[4-(2-hydroxy-2-phenyl-ethylamino)-phenyl]-ethylamino}-ethyl)-benzaldehyd
- N-[2-Hydroxy-5-(1-hydroxy-2-{2-[4-(2-hydroxy-2-phenyl-ethylamino)-phenyl]-ethylamino}-ethyl)-phenyl]-formamid
- 8-Hydroxy-5-(1-hydroxy-2-{2-[4-(6-methoxy-biphenyl-3-ylamino)-phenyl]-ethylamino}-ethyl)-1H-quinolin-2-on
- 8-Hydroxy-5-[1-hydroxy-2-(6-phenethylamino-hexylamino)-ethyl]-1H-quinolin-2-on
- 5-[2-(2-{4-[4-(2-Amino-2-methyl-propoxy)-phenylamino]-phenyl}-ethylamino)-1-hydroxy-ethyl]-8-hydroxy-1H-quinolin-2-on
- [3-(4-{6-[2-Hydroxy-2-(4-hydroxy-3-hydroxymethyl-phenyl)-ethylamino]-hexyloxy}-butyl)-5-methyl-phenyl]-harnstoff
- 4-(2-{6-[2-(2,6-Dichloro-benzyloxy)-ethoxy]-hexylamino}-1-hydroxy-ethyl)-2-hydroxymethyl-phenol
- 3-(4-{6-[2-Hydroxy-2-(4-hydroxy-3-hydroxymethyl-phenyl)-ethylamino]-hexyloxy}-butyl)-benzylsulfonamid
- 3-(3-{7-[2-Hydroxy-2-(4-hydroxy-3-hydroxymethyl-phenyl)-ethylamino]-heptyloxy}-propyl)-benzylsulfonamid
- 4-(2-{6-[4-(3-Cyclopentanesulfonyl-phenyl)-butoxy]-hexylamino}-1-hydroxy-ethyl)-2-hydroxymethyl-phenol ..
- N-Adamantan-2-yl-2-(3-{2-[2-hydroxy-2-(4-hydroxy-3-hydroxymethyl-phenyl)-ethylamino]-propyl}-phenyl)-acetamid
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydrop-toluolsulfonat.

Als Anticholinergika gelangen hierbei vorzugsweise Verbindungen zur Anwendung, die ausgewählt sind aus der Gruppe bestehend aus Tiotropiumsalzen, bevorzugt das Bromidsalz, Oxitropiumsalzen, bevorzugt das Bromidsalz, Flutropiumsalzen, bevorzugt das Bromidsalz, Ipratropiumsalzen, bevorzugt das Bromidsalz, Glycopyrroniumsalzen, bevorzugt das Bromidsalz, Trospiumsalzen, bevorzugt das Chloridsalz, Tolterodin. In den vorstehend genannten Salzen stellen die Kationen die pharmakologisch aktiven Bestandteile dar. Als Anionen können die vorstehend genannten Salze bevorzugt enthalten Chlorid, Bromid, Iodid, Sulfat, Phosphat, Methansulfonat, Nitrat, Maleat, Acetat, Citrat, Fumarat, Tartrat, Oxalat, Succinat, Benzoat oder p-Toluolsulfonat, wobei Chlorid, Bromid, Iodid, Sulfat, Methansulfonat oder p-Toluolsulfonat als Gegenionen bevorzugt sind. Von allen Salzen sind die Chloride, Bromide, Iodide und Methansulfonate besonders bevorzugt.

Ebenfalls bevorzugte Anticholinergika sind ausgewählt aus den Salzen der Formel **AC-1** 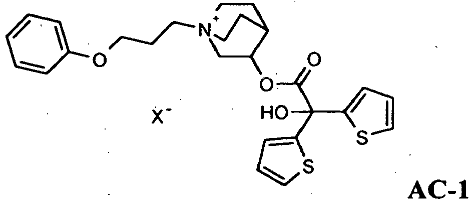 worin X - ein einfach negativ geladenes Anion, bevorzugt ein Anion ausgewählt aus der Gruppe bestehend aus Fluorid, Chlorid, Bromid, Iodid, Sulfat, Phosphat, Methansulfonat, Nitrat, Maleat, Acetat, Citrat, Fumarat, Tartrat, Oxalat, Succinat, Benzoat und p-Toluolsulfonat, bevorzugt ein einfach negativ geladenes Anion, besonders bevorzugt ein Anion ausgewählt aus der Gruppe bestehend aus Fluorid, Chlorid, Bromid, Methansulfonat und p-Toluolsulfonat, insbesondere bevorzugt Bromid, bedeutet gegebenenfalls in Form ihrer Racemate, Enantiomere oder Hydrate. Von besonderer Bedeutung sind solche Arzneimittelkombinationen, die die Enantiomere der Formel **AC-1-en** 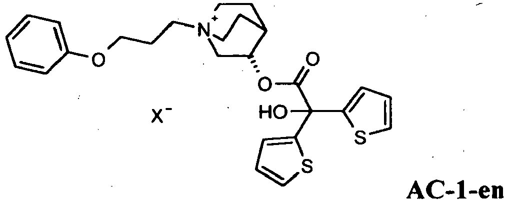 enthalten, worin X - die vorstehend genannten Bedeutungen aufweisen kann. Weiterhin bevorzugte Anticholinergika sind ausgewählt aus den Salzen der Formel **AC-2** 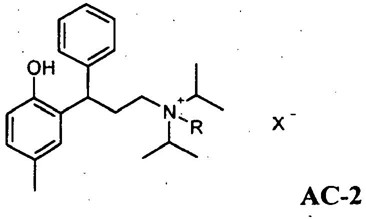 worin R entweder Methyl oder Ethyl bedeuten und worin X - die vorstehend genannte Bedeutungen aufweisen kann. In einer alternativen Ausführungsform kann die Verbindung der Formel **AC-2** auch in Form der freien Base **AC-2-base** vorliegen.

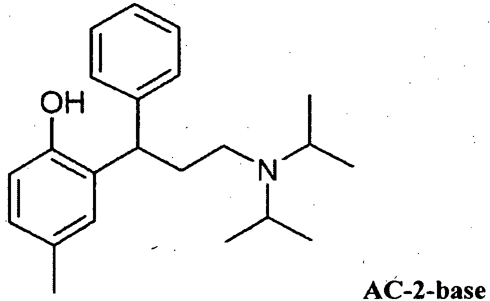

Weiterhin genannte Verbindungen sind:
- 2,2-Diphenylpropionsäuretropenolester-Methobromid
- 2,2-Diphenylpropionsäurescopinester-Methobromid
- 2-Fluor-2,2-Dipheny(essigsäurescopinester-Methobromid
- 2-Fluor-2,2-Diphenylessigsäuretropenolester-Methobromid
- 3,3',4,4'-Tetrafluorbenzilsäuretropenolester-Methobromid
- 3,3',4,4'-Tetrafluorbenzilsäurescopinester-Methobromid
- 4,4'-Difluorbenzilsäuretropenolester-Methobromid
- 4,4'-Difluorbenzilsäurescopinester-Methobromid
- 3,3'-Difluorbenzilsäuretropenolester-Methobromid
- 3,3'-Difluorbenzilsäurescopinester-Methobromid
- 9-Hydroxy-fluoren-9-carbonsäuretropenolester-Methobromid
- 9-Fluor-fluoren-9-carbonsäuretropenolester-Methobromid
- 9-Hydroxy-fluoren-9-carbonsäurescopinester-Methobromid
- 9-Fluor-fluoren-9-carbonsäurescopinester-Methobromid
- 9-Methyl-fluoren-9-carbonsäuretropenolester-Methobromid
- 9-Methyl-fluoren-9-carbonsäurescopinester-Methobromid
- Benzilsäurecyclopropyltropinester-Methobromid
- 2,2-Diphenylpropionsäurecyclopropyltropinester-Methobromid
- 9-Hydroxy-xanthen-9-carbonsäurecyclopropyltropinester-Methobromid
- 9-Methyl-fluoren-9-carbonsäurecyclopropyltropinester-Methobromid
- 9-Methyl-xanthen-9-carbonsäurecyclopropyltropinester-Methobromid
- 9-Hydroxy-fluoren-9-carbonsäurecyclopropyltropinester-Methobromid
- 4,4'-Difluorbenzilsäuremethylestercyclopropyltropinester-Methobromid
- 9-Hydroxy-xanthen-9-carbonsäuretropenolester-Methobromid
- 9-Hydroxy-xanthen-9-carbonsäurescopinester-Methobromid
- 9-Methyl-xanthen-9-carbonsäuretropenolester-Methobromid
- 9-Methyl-xanthen-9-carbonsäurescopinester-Methobromid
- 9-Ethyl-xanthen-9-carbonsäuretropenolester-Methobromid
- 9-Difluormethyl-xanthen-9-carbonsäuretropenolester-Methobromid
- 9-Hydroxymethyl-xanthen-9-carbonsäurescopinester-Methobromid

Die vorstehend genannten Verbindungen sind im Rahmen der vorliegenden Erfindung auch als Salze einsetzbar, in denen statt des Methobromids, die Salze Metho-X zur Anwendung gelangen, wobei X die vorstehend für X⁻ genannten Bedeutungen haben kann.

Als Corticosteroide gelangen hierbei vorzugsweise Verbindungen zur Anwendung, die ausgewählt sind aus der Gruppe bestehend aus Beclomethason, Betamethason, Budesonid, Butixocort, Ciclesonid, Deflazacort, Dexamethason, Etiprednol, Flunisolid, Fluticason, Loteprednol, Mometason, Prednisolon, Prednison, Rofleponid, Triamcinolon, RPR-106541, NS-126, ST-26 und
- 6,9-Difluor-17-[(2-furanylcarbonyl)oxy]-11-hydroxy-16-methyl-3-oxo-androsta-1,4-dien-17-carbothionsäure (S)-fluoromethylester
- 6,9-Difluor-11-hydroxy-16-methyl-3-oxo-17-propionyloxy-androsta-1,4-dien-17-carbothionsäure (S)-(2-oxo-tetrahydro-furan-3S-yl)ester,
- 6α,9α-difluoro-11β-hydroxy-16α-methyl-3-oxo-17α-(2,2,3,3-tertamethylcyclopropylcarbonyl)oxy-androsta-1,4-diene-17β-carbonsäure cyanomethyl ester
gegebenenfalls in Form ihrer Racemate, Enantiomere oder Diastereomere und gegebenenfalls in Form ihrer Salze und Derivate, ihrer Solvate und/oder Hydrate. Jede Bezugnahme auf Steroide schließt eine Bezugnahme auf deren gegebenenfalls existierende Salze oder Derivate, Hydrate oder Solvate mit ein. Beispiele möglicher Salze und Derivate der Steroide können sein: Alkalisalze, wie beispielsweise Natrium- oder Kaliumsalze, Sulfobenzoate, Phosphate, Isonicotinate, Acetate, Dichloroacetate, Propionate, Dihydrogenphosphate, Palmitate, Pivalate oder auch Furoate..

Als PDE4-Inhibitoren gelangen hierbei vorzugsweise Verbindungen zur Anwendung, die ausgewählt sind aus der Gruppe bestehend aus Enprofyllin, Theophyllin, Roflumilast, Ariflo (Cilomilast), Tofimilast, Pumafentrin, Lirimilast, Arofyllin, Atizoram, D-4418, Bay-198004, BY343, CP-325,366, D-4396 (Sch-351591), AWD-12-281 (GW-842470), NCS-613, CDP-840, D-4418, PD-168787, T-440, T-2585, V-11294A, C1-1018, CDC-801, CDC-3052, D-22888, YM-58997, Z-15370 und
- N-(3,5-Dichloro-1-oxo-pyridin-4-yl)-4-difluormethoxy-3-cyclopropylmethoxybenzamid
- (-)p-[(4aR*,10bS*)-9-Ethoxy-1,2,3,4,4a,10b-hexahydro-8-methoxy-2-methylbenzo[s][1,6]naphthyridin-6-yl]-N,N-diisopropylbenzamid
- (R)-(+)-1-(4-Brombenzyl)-4-[(3-cyclopentyloxy)-4-methoxyphenyl]-2-pyrrolidon
- 3-(Cyclopentyloxy-4-methoxyphenyl)-1-(4-N'-[N-2-cyano-S-methyl-isothioureido]benzyl)-2-pyrrolidon
- cis[4-Cyano-4-(3-cyclopentyloxy-4-methoxyphenyl)cyclohexan-1-carbonsäure]
- 2-carbomethoxy-4-cyano-4-(3-cyclopropylmethoxy-4-difluoromethoxy-phenyl)cyclohexan-1-on
- cis[4-Cyano-4-(3-cyclopropylmethoxy-4-difluormethoxyphenyl)cyclohexan-1-ol]
- (R)-(+)-Ethyl[4-(3-cyclopentyloxy-4-methoxyphenyl)pyrrolidin-2-yliden]acetat
- (S)-(-)-Ethyl[4-(3-cyclopentyloxy-4-methoxyphenyl)pyrrolidin-2-yliden]acetat
- 9-Cyclopentyl-5,6-dihydro-7-ethyl-3-(2-thienyl)-9*H*-pyrazolo[3,4-c]-1,2,4-triazolo[4,3-a]pyridin
- 9-Cyclopentyl-5,6-dihydro-7-ethyl-3-(*tert*-butyl)-9*H*-pyrazolo[3,4-c]-1,2,4-triazolo[4,3-a]pyridin
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydrop-toluolsulfonat.

Als LTD4-Antagonisten gelangen hierbei vorzugsweise Verbindungen zur Anwendung, die ausgewählt sind aus der Gruppe bestehend aus Montelukast, Pranlukast, Zafirlukast, MCC-847 (ZD-3523), MN-001, MEN-91507 (LM-1507), VUF-5078, VUF-K-8707, L-733321 und
- 1-(((R)-(3-(2-(6,7-Difluor-2-quinolinyl)ethenyl)phenyl)-3-(2-(2-hydroxy-2-propyl)phenyl)thio)methylcyclopropan-essigsäure,
- 1-(((1(R)-3(3-(2-(2,3-Dichlorthieno[3,2-b]pyridin-5-yl)-(E)-ethenyl)phenyl)-3-(2-(1-hydroxy-1-methylethyl)phenyl)-propyl)thio)methyl)cyclopropanessigsäure
- [2-[[2-(4-tert-Butyl-2-thiazolyl)-5-benzofuranyl]oxymethyl]phenyl]-essigsäure
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat. Unter Salzen oder Derivaten zu deren Bildung die LTD4-Antagonisten gegebenenfalls in der Lage sind, werden beispielsweise verstanden: Alkalisalze, wie beispielsweise Natrium- oder Kaliumsalze, Erdalkalisalze, Sulfobenzoate, Phosphate, Isonicotinate, Acetate, Propionate, Dihydrogenphosphate, Palmitate, Pivalate oder auch Furoate.

Als EGFR-Hemmer gelangen hierbei vorzugsweise Verbindungen zur Anwendung, die ausgewählt sind aus der Gruppe bestehend aus Cetuximab, Trastuzumab, ABX-EGF, Mab ICR-62 und
- 4-[(3-Chlor-4-fluorphenyl)amino]-6-{[4-(morpholin-4-yl)-1-oxo-2-buten-1-yl]amino}-7-cyclopropylmethoxy-chinazolin
- 4-[(3-Chlor-4-fluorphenyl)amino]-6-{[4-(N,N-diethylamino)-1-oxo-2-buten-1-yl]amino}-7-cyclopropylmethoxy-chinazolin
- 4-[(3-Chlor-4-fluorphenyl)amino]-6-{[4-(N,N-dimethylamino)-1-oxo-2-buten-1-yl]amino}-7-cyclopropylmethoxy-chinazolin
- 4-[(R)-(1-Phenyl-ethyl)amino]-6-{[4-(morpholin-4-yl)-1-oxo-2-buten-1-yl]amino}-7-cyclopentyloxy-chinazolin
- 4-[(3-Chlor-4-fluor-phenyl)amino]-6-{[4-((R)-6-methyl-2-oxo-morpholin-4-yl)-1-oxo-2-buten-1-yl]amino}-7-cyclopropylmethoxy-chinazolin
- 4-[(3-Chlor-4-fluor-phenyl)amino]-6-{[4-((R)-6-methyl-2-oxo-morpholin-4-yl)-1-oxo-2-buten-1-yl]amino}-7-[(S)-(tetrahydrofuran-3-yl)oxy]-chinazolin
- 4-[(3-Chlor-4-fluor-phenyl)amino]-6-{[4-((R)-2-methoxymethyl-6-oxo-morpholin-4-yl)-1-oxo-2-buten-1-yl]amino}-7-cyclopropylmethoxy-chinazolin
- 4-[(3-Chlor-4-fluor-phenyl)amino]-6-[2-((S)-6-methyl-2-oxo-morpholin-4-yl)-ethoxy]-7-methoxy-chinazolin
- 4-[(3-Chlor-4-fluorphenyl)amino]-6-({4-[N-(2-methoxy-ethyl)-N-methyl-amino]-1-oxo-2-buten-1-yl}amino)-7-cyclopropylmethoxy-chinazolin
- 4-[(3-Chlor-4-fluorphenyl)amino]-6-{[4-(N,N-dimethylamino)-1-oxo-2-buten-1-yl]amino}-7-cyclopentyloxy-chinazolin
- 4-[(R)-(1-Phenyl-ethyl)amino]-6-{[4-(N,N-bis-(2-methoxy-ethyl)-amino)-1-oxo-2-buten-1-yl]amino}-7-cydopropylmethoxy-chinazolin
- 4-[(R)-(1-Phenyl-ethyl)amino]-6-({4-[N-(2-methoxy-ethyl)-N-ethyl-amino]-1-oxo-2-buten-1-yl}amino)-7-cyclopropylmethoxy-chinazolin
- 4-[(R)-(1-Phenyl-ethyl)amino]-6-({4-[N-(2-methoxy-ethyl)-N-methyl-amino]-1-oxo-2-buten-1-yl}amino)-7-cyclopropylmethoxy-chinazolin
- 4-[(R)-(1-Phenyl-ethyl)amino]-6-({4-[N-(tetrahydropyran-4-yl)-N-methyl-amino]-1-oxo-2-buten-1-yl}amino)-7-cyclopropylmethoxy-chinazolin
- 4-[(3-Chlor-4-fluorphenyl)amino]-6-{[4-(N,N-dimethylamino)-1-oxo-2-buten-1-yl]amino}-7-((R)-tetrahydrofuran-3-yloxy)-chinazolin
- 4-[(3-Chlor-4-fluorphenyl)amino]-6-{[4-(N,N-dimethylamino)-1-oxo-2-buten-1-yl]amino}-7-((S)-tetrahydrofuran-3-yloxy)-chinazolin
- 4-[(3-Chlor-4-fluorphenyl)amino]-6-({4-[N-(2-methoxy-ethyl)-N-methyl-amino]-1-oxo-2-buten-1-yl}amino)-7-cyclopentyloxy-chinazolin
- 4-[(3-Chlor-4-fluorphenyl)amino]-6-{[4-(N-cyclopropyl-N-methyl-amino)-1-oxo-2-buten-1-yl]amino}-7-cyclopentyloxy-chinazolin
- 4-[(3-Chlor-4-fluorphenyl)amino]-6-{[4-(N,N-dimethylamino)-1-oxo-2-buten- 1-yl]amino}-7-[(R)-(tetrahydrofuran-2-yl)methoxy]-chinazolin
- 4-[(3-Chlor-4-fluorphenyl)amino]-6-{[4-(N,N-dimethylamino)-1-oxo-2-buten-1-yl]amino}-7-[(S)-(tetrahydrofuran-2-yl)methoxy]-chinazolin
- 4-[(3-Ethinyl-phenyl)amino]-6,7-bis-(2-methoxy-ethoxy)-chinazolin
- 4-[(3-Chlor-4-fluorphenyl)amino]-7-[3-(morpholin-4-yl)-propyloxy]-6-[(vinylcarbonyl)amino]-chinazolin
- 4-[(R)-(1-Phenyl-ethyl)amino]-6-(4-hydroxy-phenyl)-7H-pyrrolo[2,3-d]pyrimidin
- 3-Cyano-4-[(3-chlor-4-fluorphenyl)amino]-6-{[4-(N,N-dimethylamino)-1-oxo-2-buten-1-yl]amino}-7-ethoxy-chinolin
- 4-{[3-Chlor-4-(3-fluor-benzyloxy)-phenyl]amino}-6-(5-{[(2-methansulfonyl-ethyl)amino]methyl}-furan-2-yl)chinazolin
- 4-[(R)-(1-Phenyl-ethyl)amino]-6-{[4-((R)-6-methyl-2-oxo-morpholin-4-yl)-1-oxo-2-buten-1-yl]amino}-7-methoxy-chinazolin
- 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin
- 4-[(3-Chlor-4-fluorphenyl)amino]-6-({4-[N,N-bis-(2-methoxy-ethyl)-amino]-1-oxo-2-buten-1-yl}amino)-7-[(tetrahydrofuran-2-yl)methoxy]-chinazolin
- 4-[(3-Ethinyl-phenyl)amino]-6-{[4-(5,5-dimethyl-2-oxo-morpholin-4-yl)-1-oxo-2-buten-1-yl]amino}-chinazolin
- 4-[(3-Chlor-4-fluor-phenyl)amino]-6-[2-(2,2-dimethyl-6-oxo-morpholin-4-yl)-ethoxy]-7-methoxy-chinazolin
- 4-[(3-Chlor-4-fluor-phenyl)amino]-6-[2-(2,2-dimethyl-6-oxo-morpholin-4-yl)-ethoxy]-7-[(R)-(tetrahydrofuran-2-yl)methoxy]-chinazolin
- 4-[(3-Chlor-4-fluor-phenyl)amino]-7-[2-(2,2-dimethyl-6-oxo-morpholin-4-yl)-ethoxy]-6-[(S)-(tetrahydrofuran-2-yl)methoxy]-chinazolin
- 4-[(3-Chlor-4-fluor-phenyl)amino]-6-{2-[4-(2-oxo-morpholin-4-yl)-piperidin-1-yl]-ethoxy}-7-methoxy-chinazolin
- 4-[(3-Chlor-4-fluor-phenyl)amino]-6-[1-(tert.-butyloxycarbonyl)-piperidin-4-yloxy]-7-methoxy-chinazolin
- 4-[(3-Chlor-4-fluor-phenyl)amino]-6-(trans-4-amino-cyclohexan-1-yloxy)-7-methoxy-chinazolin
- 4-[(3-Chlor-4-fluor-phenyl)amino]-6-(trans-4-methansulfonylamino-cyclohexan-1-yloxy)-7-methoxy-chinazolin
- 4-[(3-Chlor-4-fluor-phenyl)amino]-6-(tetrahydropyran-3-yloxy)-7-methoxy-chinazolin
- 4-[(3-Chlor-4-fluor-phenyl)amino]-6-(1-methyl-piperidin-4-yloxy)-7-methoxy-chinazolin
- 4-[(3-Chlor-4-fluor-phenyl)amino]-6-{1-[(morpholin-4-yl)carbonyl]-piperidin-4-yloxy}-7-methoxy-chinazolin
- 4-[(3-Chlor-4-fluor-phenyl)amino]-6-{1-[(methoxymethyl)carbonyl]-piperidin-4-yloxy}-7-methoxy-chinazolin
- 4-[(3-Chlor-4-fluor-phenyl)amino]-6-(piperidin-3-yloxy)-7-methoxy-chinazolin
- 4-[(3-Chlor-4-fluor-phenyl)amino]-6-[1-(2-acetylamino-ethyl)-piperidin-4-yloxy]-7-methoxy-chinazolin
- 4-[(3-Chlor-4-fluor-phenyl)amino]-6-(tetrahydropyran-4-yloxy)-7-ethoxy-chinazolin
- 4-[(3-Chlor-4-fluor-phenyl)amino]-6-((S)-tetrahydrofuran-3-yloxy)-7-hydroxy-chinazolin
- 4-[(3-Chlor-4-fluor-phenyl)amino]-6-(tetrahydropyran-4-yloxy)-7-(2-methoxy-ethoxy)-chinazolin
- 4-[(3-Chlor-4-fluor-phenyl)amino]-6-{trans-4-[(dimethylamino)sulfonylamino]-cyclohexan-1-yloxy}-7-methoxy-chinazolin
- 4-[(3-Chlor-4-fluor-phenyl)amino]-6-{trans-4-[(morpholin-4-yl)carbonylamino]-cyclohexan-1-yloxy}-7-methoxy-chinazolin
- 4-[(3-Chlor-4-fluor-phenyl)amino]-6-{trans-4-[(morpholin-4-yl)sulfonylamino]-cyclohexan-1-yloxy}-7-methoxy-chinazolin
- 4-[(3-Chlor-4-fluor-phenyl)amino]-6-(tetrahydropyran-4-yloxy)-7-(2-acetylamino-ethoxy)-chinazolin
- 4-[(3-Chlor-4-fluor-phenyl)amino]-6-(tetrahydropyran-4-yloxy)-7-(2-methansulfonylamino-ethoxy)-chinazolin
- 4-[(3-Chlor-4-fluor-phenyl)amino]-6-{1-[(piperidin-1-yl)carbonyl]-piperidin-4-yloxy}-7-methoxy-chinazolin
- 4-[(3-Chlor-4-fluor-phenyl)amino]-6-(1-aminocarbonylmethyl-piperidin-4-yloxy)-7-meₜhoxy-chinazolin
- 4-[(3-Chlor-4-fluor-phenyl)amino]-6-(cis-4-{N-[(tetrahydropyran-4-yl)carbonyl]-N-methyl-amino}-cyclohexan-1-yloxy)-7-methoxy-chinazolin
- 4-[(3-Chlor-4-fluor-phenyl)amino]-6-(cis-4-{N-[(morpholin-4-yl)carbonyl]-N-methyl-amino}-cyclohexan-1-yloxy)-7-methoxy-chinazolin
- 4-[(3-Chlor-4-fluor-phenyl)amino]-6-(cis-4-{N-[(morpholin-4-yl)sulfonyl]-N-methyl-amino}-cyclohexan-1-yloxy)-7-methoxy-chinazolin
- 4-[(3-Chlor-4-fluor-phenyl)amino]-6-(trans-4-ethansulfonylamino-cyclohexan-1-yloxy)-7-methoxy-chinazolin
- 4-[(3-Chlor-4-fluor-phenyl)amino]-6-(1-methansulfonyl-piperidin-4-yloxy)-7-ethoxy-chinazolin
- 4-[(3-Chlor-4-fluor-phenyl)amino]-6-(1-methansulfonyl-piperidin-4-yloxy)-7-(2-methoxy-ethoxy)-chinazolin
- 4-[(3-Chlor-4-fluor-phenyl)amino]-6-[1-(2-methoxy-acetyl)-piperidin-4-yloxy]-7-(2-methoxy-ethoxy)-chinazolin
- 4-[(3-Chlor-4-fluor-phenyl)amino]-6-(cis-4-acetylamino-cyclohexan-1-yloxy)-7-methoxy-chinazolin
- 4-[(3-Ethinyl-phenyl)amino]-6-[1-(tert.-butyloxycarbonyl)-piperidin-4-yloxy]-7-methoxy-chinazolin
- 4-[(3-Ethinyl-phenyl)amino]-6-(tetrahydropyran-4-yloxy]-7-methoxy-chinazolin
- 4-[(3-Chlor-4-fluor-phenyl)amino]-6-(cis-4-{N-[(piperidin-1-yl)carbonyl]-N-methyl-amino}-cyclohexan-1-yloxy)-7-methoxy-chinazolin
- 4-[(3-Chlor-4-fluor-phenyl)amino]-6-(cis-4-{N-[(4-methyl-piperazin-1-yl)carbonyl]-N-methyl-amino}-cyclohexan-1-yloxy)-7-methoxy-chinazolin
- 4-[(3-Chlor-4-fluor-phenyl)amino]-6-{cis-4-[(morpholin-4-yl)carbonyiamino]-cyclohexan-1-yloxy}-7-methoxy-chinazolin
- 4-[(3-Chlor-4-fluor-phenyl)amino]-6-{1-[2-(2-oxopyrrolidin-1-yl)ethyl]-piperidin-4-yloxy}-7-methoxy-chinazolin
- 4-[(3-Chlor-4-fluor-phenyl)amino]-6-{1-[(morpholin-4-yl)carbonyl]-piperidin-4-yloxy}-7-(2-methoxy-ethoxy)-chinazolin
- 4-[(3-Ethinyl-phenyl)amino]-6-(1-acetyl-piperidin-4-yloxy)-7-methoxy-chinazolin
- 4-[(3-Ethinyl-phenyl)amino]-6-(1-methyl-piperidin-4-yloxy)-7-methoxy-chinazolin
- 4-[(3-Ethinyl-phenyl)amino]-6-(1-methansulfonyl-piperidin-4-yloxy)-7-methoxy-chinazolin
- 4-[(3-Chlor-4-fluor-phenyl)amino]-6-(1-methyl-piperidin-4-yloxy)-7(2-methoxy-ethoxy)-chinazolin
- 4-[(3-Chlor-4-fluor-phenyl)amino]-6-(1-isopropyloxycarbonyl-piperidin-4-yloxy)-7-methoxy-chinazolin
- 4-[(3-Chlor-4-fluor-phenyl)amino]-6-(cis-4-methylamino-cyclohexan-1-yloxy)-7-methoxy-chinazolin
- 4-[(3-Chlor-4-fluor-phenyl)amino]-6-{cis-4-[N-(2-methoxy-acetyl)-N-methyl-amino]-cyclohexan-1-yloxy}-7-methoxy-chinazolin
- 4-[(3-Ethinyl-phenyl)amino]-6-(piperidin-4-yloxy)-7-methoxy-chinazolin
- 4-[(3-Ethinyl-phenyl)amino]-6-[1-(2-methoxy-acetyl)-piperidin-4-yloxy]-7-methoxy-chinazolin
- 4-[(3-Ethinyl-phenyl)amino]-6-{1-[(morpholin-4-yl)carbonyl]-piperidin-4-yloxy}-7-methoxy-chinazolin
- 4-[(3-Chlor-4-fluor-phenyl)amino]-6-{1-[(cis-2,6-dimethyl-morpholin-4-yl)carbonyl]-piperidin-4-yloxy}-7-methoxy-chinazolin
- 4-[(3-Chlor-4-fluor-phenyl)amino]-6-{1-[(2-methyl-morpholin-4-yl)carbonyl]-piperidin-4-yloxy}-7-methoxy-chinazolin
- 4-[(3-Chlor-4-fluor-phenyl)amino]-6-{1-[(S,S)-(2-oxa-5-aza-bicyclo[2.2.1]hept-5-yl)carbonyl]-piperidin-4-yloxy}-7-methoxy-chinazolin
- 4-[(3-Chlor-4-fluor-phenyl)amino]-6-{1-[(N-methyl-N-2-methoxyethyl-amino)carbonyl]-piperidin-4-yloxy}-7-methoxy-chinazolin
- 4-[(3-Chlor-4-fluor-phenyl)amino]-6-(1-ethyl-piperidin-4-yloxy)-7-methoxy-chinazolin
- 4-[(3-Chlor-4-fluor-phenyl)amino]-6-{1-[(2-methoxyethyl)carbonyl]-piperidin-4-yloxy}-7-methoxy-chinazolin
- 4-[(3-Chlor-4-fluor-phenyl)amino]-6-{1-[(3-methoxypropyl-amino)-carbonyl]-piperidin-4-yloxy}-7-methoxy-chinazolin
- 4-[(3-Chlor-4-fluor-phenyl)amino]-6-[cis-4-(N-methansulfonyl-N-methyl-amino)-cyclohexan-1-yloxy]-7-methoxy-chinazolin
- 4-[(3-Chlor-4-fluor-phenyl)amino]-6-[cis-4-(N-acetyl-N-methyl-amino)-cyclohexan-1-yloxy]-7-methoxy-chinazolin
- 4-[(3-Chlor-4-fluor-phenyl)amino]-6-(trans-4-methylamino-cyclohexan-1-yloxy)-7-methoxy-chinazolin
- 4-[(3-Chlor-4-fluor-phenyl)amino]-6-[trans-4-(N-methansulfonyl-N-methyl-amino)-cyclohexan-1-yloxy]-7-methoxy-chinazolin
- 4-[(3-Chlor-4-fluor-phenyl)amino]-6-(trans-4-dimethylamino-cyclohexan-1-yloxy)-7-methoxy-chinazolin
- 4-[(3-Chlor-4-fluor-phenyl)amino]-6-(trans-4-{N-[(morpholin-4-yl)carbonyl]-N-methyl-amino}-cyclohexan-1-yloxy)-7-methoxy-chinazolin
- 4-[(3-Chlor-4-fluor-phenyl)amino]-6-[2-(2,2-dimethyl-6-oxo-morpholin-4-yl)-ethoxy]-7-[(S)-(tetrahydrofuran-2-yl)methoxy]-chinazolin
- 4-[(3-Chlor-4-fluor-phenyl)amino]-6-(1-methansulfonyl-piperidin-4-yloxy)-7-methoxy-chinazolin
- 4-[(3-Chlor-4-fluor-phenyl)amino]-6-(1-cyano-piperidin-4-yloxy)-7-methoxy-chinazolin
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydrop-toluolsulfonat.

Als Dopamin-Agonisten gelangen hierbei vorzugsweise Verbindungen zur Anwendung, die ausgewählt sind aus der Gruppe bestehend aus Bromocriptin, Cabergolin, Alpha-Dihydroergocryptin, Lisurid, Pergolid, Pramipexol, Roxindol, Ropinirol, Talipexol, Tergurid und Viozan, gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als H1-Antihistaminika gelangen hierbei vorzugsweise Verbindungen zur Anwendung, die ausgewählt sind aus der Gruppe bestehend aus Epinastin, Cetirizin, Azelastin, Fexofenadin, Levocabastin, Loratadin, Mizolastin, Ketotifen, Emedastin, Dimetinden, Clemastin, Bamipin, Cexchlorpheniramin, Pheniramin, Doxylamin, Chlorphenoxamin, Dimenhydrinat, Diphenhydramin, Promethazin, Ebastin, Desloratidin und Meclozin, gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Außerdem können inhalierbare Makromoleküle verwendet werden, wie in EP 1 003 478 A1 oder CA 2297174 A1, offenbart.

Weiterhin kann die Verbindung aus der Gruppe der Derivate von Mutterkornalkaloiden, der Triptane, der CGRP-Hemmern, der Phosphodiesterase-V-Hemmer stammen, gegebenenfalls in Form ihrer Racemate, Enantiomere oder Diastereomere, gegebenenfalls in Form ihrer pharmakologisch verträglichen Säureadditionssalze, ihrer Solvate und/oder Hydrate.

Als Derivate der Mutterkornalkaloide: Dihydroergotamin, Ergotamin.

**Bezugszeichenliste**

| | | | |
|---|---|---|---|
| 1 | Inhalator | 14 | Betätigungseinrichtung |
| 2 | Formulierung | 15 | Feder |
| 3 | Reservoir | 16 | Kolben |
| 4 | Dosiereinrichtung | 17 | Kolbenführung |
| 5 | Ausgabekanal | 18 | Ventileinrichtung |
| 6 | Auslaß | 19 | Verbindungskanal |
| 7 | Dosierkammer | 20 | erster Anschlußkanal |
| 8 | Wandung | 21 | zweiter Anschlußkanal |
| 9 | Zuführleitung | 22 | Zuluftkanal |
| 10 | Zuführkammer | 23 | Rückstellfeder |
| 11 | Pumpeinrichtung | 24 | Dosierkammerteil |
| 12 | Pumpenkolben | 25 | Dosierkammerteil |
| 13 | Pumpenraum | 26 | Dosierkammerteil |

## Patentansprüche

1. Dosiereinrichtung (4), vorzugsweise für einen Inhalator (1), zur Dosierung einer Kleinmenge einer insbesondere pulverförmigen Formulierung (2),
mit einem Reservoir (3) für die Formulierung (2),
mit einer Dosierkammer (7) zur Aufnahme der vom Reservoir (3) bereitgestellten Formulierung (2), wobei die Dosierkammer (7) eine zumindest bereichsweise gasdurchlässige Wandung (8) aufweist, so daß die Formulierung (2) durch Unterdruck bzw. einen Luftstrom in die Dosierkammer (7) aufnehmbar und durch Überdruck bzw. einen Luftstrom aus der Dosierkammer (7) wieder abgebbar ist,
wobei die Dosiereinrichtung (4) eine Pumpeinrichtung (11) zum abwechselnden Erzeugen des Unterdrucks bzw. Luftstroms zum Aufnehmen der Formulierung (2) in die Dosierkammer (7) und des Überdrucks bzw. Luftstroms zur Abgabe der Formulierung (2) aus der Dosierkammer (7) aufweist, und wobei die Dosiereinrichtung (4) einen die Dosierkammer (7) tragenden oder bildenden Kolben (16) aufweist, der zwischen einer Aufnahmeposition zur Aufnahme der Formulierung (2) in die Dosierkammer (7) und einer Abgabeposition zur Abgabe der Formulierung (2) aus der Dosierkammer (7) bewegbar ist
**dadurch gekennzeichnet, dass**
die Wandung (8) eine poröse Wandung ist, durch die hindurch ein Luftstrom vom Reservoir (3) in die Dosierkammer (7) erzeugt werden kann und wodurch die Formulierung (2) in die Dosierkammer (7) gefördert und die Dosierkammer (7) mit der Formulierung (2) gefüllt wird.

2. Dosiereinrichtung nach Anspruch 1, **dadurch gekennzeichnet, daß** die Pumpeinrichtung (11) manuell betätigbar ist.

3. Dosiereinrichtung nach Anspruch 1, **dadurch gekennzeichnet, daß** die Dosiereinrichtung (4) eine Pumpeinrichtung (11) zum Erzeugen des Unterdrucks und/oder Überdrucks bzw. Luftstroms oder der Luftströme aufweist.

4. Dosiereinrichtung nach Anspruch 3, **dadurch gekennzeichnet, daß** der Kolben (16) die Pumpeinrichtung (11) bildet oder mit dieser gekoppelt ist.

5. Dosiereinrichtung nach Anspruch 3 oder 4, **dadurch gekennzeichnet, daß** der Kolben (16) eine Ventileinrichtung (18) insbesondere der Pumpeinrichtung (11) bildet und/oder steuert.

6. Dosiereinrichtung nach einem der Ansprüche 3 bis 5, **dadurch gekennzeichnet, daß** die Dosiereinrichtung (4) eine gemeinsame Betätigungseinrichtung (14) für den Kolben (16) und die Pumpeinrichtung (11) aufweist.

7. Dosiereinrichtung nach einem der Ansprüche 2 bis 6, **dadurch gekennzeichnet, daß** der Kolben (16) manuell betätigbar ist.

8. Dosiereinrichtung nach einem der Ansprüche 2 bis 7, **dadurch gekennzeichnet, daß** sich der Kolben (16) durch das Reservoir (3) erstreckt.

9. Dosiereinrichtung nach einem der Ansprüche 2 bis 8, **dadurch gekennzeichnet, daß** dem Kolben (16) ein Mittel zur Lockerung der Formulierung (2) im Reservoir (3) bei Bewegung des Kolbens (16) zugeordnet ist.

10. Dosiereinrichtung nach einem der Ansprüche 2 bis 9, **dadurch gekennzeichnet, daß** die Dosiereinrichtung (4) eine dem Kolben (16) zugeordnete Feder (23) vorzugsweise zur Rückstellung des Kolbens (16) insbesondere in die Aufnahme- oder Ausgabeposition aufweist.

11. Dosiereinrichtung nach den Ansprüchen 9 und 10, **dadurch gekennzeichnet, daß** die Feder (23) das Mittel zur Lockerung bildet

12. Dosiereinrichtung nach Anspruch 10 oder 11, **dadurch gekennzeichnet, daß** die Feder (23) den Kolben (16) umgibt.

13. Dosiereinrichtung nach einem der Ansprüche 10 bis 12, **dadurch gekennzeichnet, daß** sich die Feder (23) in oder durch das Reservoir (3) erstreckt.

14. Dosiereinrichtung (4)nach einem dervoranstehenden Ansprüche, **dadurch gekennzeichnet, daß** die Größe der Dosierkammer (7) einstellbar ist.

## Claims

1. Dosing device (4), preferably for an inhaler (1), for dosing a small amount of an in particular powdered formulation (2),
having a reservoir (3) for the formulation (2),
having a dosing chamber (7) for receiving formulation (2) supplied from the reservoir (3), wherein the dosing chamber (7) has a wall (8) that is gas-permeable at least in parts, so that the formulation (2) can be taken into the dosing chamber (7) by underpressure or an air current and can be expelled from the dosing chamber (7) by overpressure or an air current,
the dosing device (4) comprising a pump device (11) for alternately generating the underpressure or air current for taking the formulation (2) into the dosing chamber (7) and the overpressure or air current for expelling the formulation (2) from the dosing chamber (7), and the dosing device (4) comprising a piston (16) that carries or forms the dosing chamber (7), which is movable between an uptake position for taking the formulation (2) into the dosing chamber (7) and an expulsion position for expelling the formulation (2) from the dosing chamber (7),
**characterised in that**
the wall (8) is a porous wall through which an air current from the reservoir (3) into the dosing chamber (7) can be generated and by means of which the formulation (2) is conveyed into the dosing chamber (7) and the dosing chamber (7) is filled with the formulation (2).

2. Dosing device according to claim 1, **characterised in that** the pump device (11) can be operated manually.

3. Dosing device according to claim 1, **characterised in that** the dosing device (4) comprises a pump device (11) for generating the underpressure and/or overpressure or air current or air currents.

4. Dosing device according to claim 3, **characterised in that** the piston (16) forms the pump device (11) or is coupled thereto.

5. Dosing device according to claim 3 or 4, **characterised in that** the piston (16) forms and/or controls a valve device (18) particularly of the pump device (11).

6. Dosing device according to one of claims 3 to 5, **characterised in that** the dosing device (4) comprises a common actuating device (14) for the piston (16) and the pump device (11).

7. Dosing device according to one of claims 2 to 6, **characterised in that** the piston (16) can be operated manually.

8. Dosing device according to one of claims 2 to 7, **characterised in that** the piston (16) extends through the reservoir (3).

9. Dosing device according to one of claims 2 to 8, **characterised in that** associated with the piston (16) is a device for loosening the formulation (2) in the reservoir (3) during the movement of the piston (16).

10. Dosing device according to one of claims 2 to 9, **characterised in that** the dosing device (4) has a spring (23) associated with the piston (16) preferably for returning the piston (16) in particular to its uptake or delivery position.

11. Dosing device according to claims 9 and 10, **characterised in that** the spring (23) forms the loosening device.

12. Dosing device according to claim 10 or 11, **characterised in that** the spring (23) surrounds the piston (16).

13. Dosing device according to one of claims 10 to 12, **characterised in that** the spring (23) extends into or through the reservoir (3).

14. Dosing device according to one of the preceding claims, **characterised in that** the dosing chamber (7) is adjustable in size.

## Revendications

1. Dispositif de dosage (4), de préférence pour un inhalateur (1), servant à doser une petite quantité d'une formulation (2) en particulier pulvérulente, comprenant :
un réservoir (3) pour la formulation (2) ;
une chambre de dosage (7) servant à recevoir la formulation (2) fournie par le réservoir (3), la chambre de dosage (7) présentant une paroi (8) au moins partiellement perméable au gaz, de sorte que la formulation (2) peut être reçue dans la chambre de dosage (7) par une dépression ou un flux d'air et peut être à nouveau évacuée de la chambre de dosage (7) par une surpression ou un flux d'air aux fins de sa distribution,
le dispositif de dosage (4) présentant un dispositif pompe (11) servant à produire en alternance la dépression ou le flux d'air afin d'amener la formulation (2) dans la chambre de dosage (7) et la surpression ou le flux d'air afin d'évacuer la formulation (2) de la chambre de dosage (7) aux fins de sa distribution, et le dispositif de dosage (4) présentant un piston (16) supportant ou formant la chambre de dosage (7), lequel piston peut être déplacé entre une position de réception pour amener la formulation (2) dans la chambre de dosage (7) et une position de distribution servant à évacuer la formulation (2) de la chambre de dosage (7) aux fins de sa distribution,
**caractérisé en ce que**
la paroi (8) est une paroi poreuse, au travers de laquelle un flux d'air peut être généré depuis le réservoir (3) dans la chambre de dosage (7) et par laquelle la formulation (2) est acheminée dans la chambre de dosage (7), qui est alors remplie par la formulation (2).

2. Dispositif de dosage selon la revendication 1, **caractérisé en ce que** le dispositif pompe (11) peut être actionné manuellement.

3. Dispositif de dosage selon la revendication 1, **caractérisé en ce que** le dispositif de dosage (4) présente un dispositif pompe (11) servant à générer la dépression et/ou la surpression ou le flux d'air ou les flux d'air.

4. Dispositif de dosage selon la revendication 3, **caractérisé en ce que** le piston (16) forme le dispositif pompe (11) ou est couplé à ce dernier.

5. Dispositif de dosage selon la revendication 3 ou 4, **caractérisé en ce que** le piston (16) forme et/ou commande un dispositif de soupape (18) en particulier du dispositif pompe (11).

6. Dispositif de dosage selon l'une quelconque des revendications 3 à 5, **caractérisé en ce que** le dispositif de dosage (4) présente un dispositif d'actionnement (14) commun pour le piston (16) et le dispositif pompe (11).

7. Dispositif de dosage selon l'une quelconque des revendications 2 à 6, **caractérisé en ce que** le piston (16) peut être actionné manuellement.

8. Dispositif de dosage selon l'une quelconque des revendications 2 à 7, **caractérisé en ce que** le piston (16) s'étend à travers le réservoir (3).

9. Dispositif de dosage selon l'une quelconque des revendications 2 à 8, **caractérisé en ce qu'**un moyen servant à relâcher la formulation (2) dans le réservoir (3) lors du mouvement du piston (16) est associé au piston (16).

10. Dispositif de dosage selon l'une quelconque des revendications 2 à 9, **caractérisé en ce que** le dispositif de dosage (4) présente un ressort (23) associé au piston (16) servant de préférence à ramener le piston (16) en particulier dans la position de réception ou dans la position de distribution.

11. Dispositif de dosage selon les revendications 9 et 10, **caractérisé en ce que** le ressort (23) forme le moyen servant au relâchement.

12. Dispositif de dosage selon la revendication 10 ou 11, **caractérisé en ce que** le ressort (23) entoure le piston (16).

13. Dispositif de dosage selon l'une quelconque des revendications 10 à 12, **caractérisé en ce que** le ressort (23) s'étend dans le réservoir (3) ou à travers ce dernier.

14. Dispositif de dosage (4) selon l'une quelconque des revendications précédentes, **caractérisé en ce que** la taille de la chambre de dosage (7) peut être réglée.
